# EUROPEAN PATENT SPECIFICATION

(11) **EP 1 517 682 B1**
(45) Date of publication and mention of the grant of the patent: **02.11.2006**
(21) Application number: 03761592.9
(22) Date of filing: 25.06.2003
(51) Int. Cl.: A61K 31/195, A61K 31/425, A61K 31/415, A61K 38/55

(54) **PHARMACEUTICAL FORMULATION COMPRISING NON-PEPTIDE RENIN INHIBITOR AND SURFACTANT**
PHARMAZEUTISCHE FORMULIERUNG MIT EINEM NICHTPEPTIDISCHEN RENIN-HEMMER UND SURFACTANT
FORMULATION PHARMACEUTIQUE COMPRENANT UN INHIBITEUR DE RENINE NON PEPTIDIQUE ET UN TENSIOACTIF

(30) Priority: 28.06.2002 CH 112402
(43) Date of publication of application: 30.03.2005
(73) Proprietor: Speedel Pharma AG, 4051 Basel (CH)
(72) Inventor: RIEDL, Jutta, 79639 Grenzach-Wyhlen (DE); KANNAH, Satish, CH-4103 Bottmingen (CH); DIETERLE, Walter, 79541 Lörrach (DE)
(74) Representative: Maué, Paul Georg
(86) International application number: PCT/EP2003/050266
(87) International publication number: WO 2004/002466

(56) References cited:
- EP-A- 0 031 603
- US-A- 5 523 289
- US-A1- 2001 007 663
- US-B1- 6 346 537
- LECLUYSE EDWARD L ET AL: "In vitro models for selection of development candidates. Permeability studies to define mechanisms of absorption enhancement" ADV DRUG DELIVERY REV;ADVANCED DRUG DELIVERY REVIEWS JAN 15 1997 ELSEVIER SCIENCE B.V., AMSTERDAM, NETHERLANDS, vol. 23, no. 1-3, 15 January 1997 (1997-01-15), pages 163-183, XP001155996 cited in the application
- KIM D-C ET AL: "EVALUATION OF THE BILE ACID TRANSPORTER IN ENHANCING INTESTINAL PERMEABILITY TO RENIN-INHIBITORY PEPTIDES" JOURNAL OF DRUG TARGETING, HARWOOD ACADEMIC PUBLISHERS GMBH, DE, vol. 1, no. 4, 1993, pages 347-359, XP000564343 ISSN: 1061-186X

## Description

The present invention relates to a composition consisting of a non-peptide renin inhibitor and an anionic surfactant, to oral administration forms comprising this composition, and to a method for improving the bioavailability of non-peptide renin inhibitors.

Non-peptide renin inhibitors are valuable compounds for treating high blood pressure, for example, and other cardiovascular diseases. A variety of these compounds have recently been disclosed. EP-A-0 716 077, WO 01/09083, WO 02/08172 and WO 02/02508 describe ω-phenyloctanecarboxamide derivatives which are very highly soluble in water. II Farmaco 56 (2001), pages 21-27, describes piperidine derivatives. Bioorganic & Medicinal Chemistry Letters (1996) Volume 6, pages 1589-1594; Arzneimittelforschung (1993), 43(2a), pages 260 to 262; Am. J. Hypertens. (1996), 9(6), pages 517-522 and Xenobiotica (1996), 26(3), pages 33-345 propose imidazole derivatives. Circulation (1995), 91(2), pages 330-338; Clin. Pharmacol. Ther. (St. Louis) (1995), 57(3), pages 342-348 and Tetrahedron (1999), 55(15), pages 4763-4768 describe thiazole derivatives as renin inhibitors.

Although non-peptide renin inhibitors have been known for a relatively long time and possess outstanding pharmacological properties and a very high degree of activity, they have not thus far been demonstrated to be suitable for broad therapeutic application, for example for treating high blood pressure using oral administration forms. The main reason lies in the low degree of bioavailability following oral administration, as is reported by various authors in II Farmaco 56 (2001), pages 21-27; Chemistry & Biology 2000, 7:493-504; Clin. Pharmacokinet. (1995), 29(1), pages 6-14 and Pharmac. Ther. (1994); Volume 61, pages 325-344. The low degree of oral bioavailability also still continues to restrict therapeutic application. It would therefore be extremely desirable to identify a galenic formulation which exhibits higher bioavailability and which can therefore be used to reduce the high requirement for material (high doses) in order, in this way, to provide suitable forms for oral administration. It is thereby also possible, where appropriate, to obtain an improvement in the case of active compounds which are less well tolerated in order, in this way, to make it possible to achieve a broader therapeutic application for the renin inhibitors.

The use of surfactants as wetting agents in oral drug forms is described in the literature, for example in H. Sucker, P. Fuchs, P. Speiser, Pharmazeutische Technologie, 2nd edition, Thieme 1989, page 260. It is known from other papers, such as published in Advanced Drug Delivery Reviews (1997), 23, pages 163-183, that it is also possible to use surfactants, inter alia, to improve the permeation and bioavailability of pharmaceutical active compounds; however, this effect does not occur in the case of all active compounds and the extent of the improvement is frequently very slight.

It has now been found, surprisingly, that it is possible to substantially increase the bioavailability of non-peptide renin inhibitors of formula I as defined in claim 1 which are particularly readily soluble in water if these inhibitors are mixed with anionic, surfactants and processed into oral administration forms. The effect is particularly surprising in the case of water-soluble renin inhibitors since water-soluble active compounds are not as a rule formulated in combination with surfactants. The effect is also unexpectedly high, because a substantial increase of the bioavailability has been achieved. The increase of bioavailability is so important that therapeutic application is made possible in more well-tolerated doses and/or in more attractive oral dosage forms.

The invention firstly relates to a composition according to claim 1 comprising (1) a non-peptide renin inhibitor which is of relatively high molecular weight (MW 500-800) and which is readily soluble in water and (2) at least one physiologically tolerated anionic surfactant with the quantity of readily soluble renin inhibitor being at least 10% by weight, based on the composition.

Within the context of the invention, readily soluble in water denotes that at least 1 g, preferably at least 30 g, and particularly preferably at least 100 g, of renin inhibitor are dissolved per 100 ml of water.

Within the context of the invention, non-peptide means that a renin inhibitor is not only composed of aminocarboxylic acids.

The quantity of readily soluble renin inhibitors can, for example, be from 10 to 90% by weight, preferably from 20 to 90% by weight, particularly preferably from 50 to 90% by weight, and in particular preferably from 60 to 90% by weight, based on the composition.

Non-peptide renin inhibitors are known and are described in the literature mentioned at the outset.

ω-Phenyloctanecarboxamide derivatives are described in EP-A-0 716 077, WO 01/09083, WO 02/06172 and WO 02/02508. ω-Phenyloctanecarboxamide derivatives include those of the formula I, 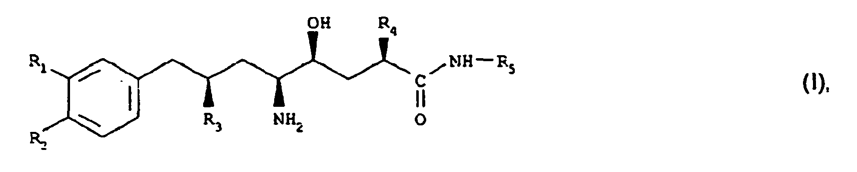 in which
R₁ and R₂ are, independently of each other, H, C₁-C₆-alkyl, C₁-C₆-haloalkyl. C₁-C₆-alkoxy, C₁-C₆-alkoxy-C₁-C₆-alkyl, or C₁-C₆-alkoxy-C₁-C₆-alkyloxy, R₃ is C₁-C₆-alkyl, R₄ is C₁-C₆-alkyl, and R₅ is C₁-C₆-alkyl, C₁-C₆-hydroxyalkyl. C₁-C₆-alkoxy-C₁-C₆-alkyl, C₁-C₆-alkanoyloxy-C₁-C₆-alkyl, C₁-C₆-aminoalkyl, C₁-C₆-alkylamino-C₁-C₆-alkyl, C₁-C₆-dialkylamino-C₁-C₆-alkyl, C₁-C₆-alkanoylamido-C₁-C₆-alkyl, HO(O)C-C₁-C₆-alkyl, C₁-C₆-alkyl-O-(O)C-C₁-C₆-alkyl, H₂N-C(O)-C₁-C₆-alkyl, C₁-C₆-alkyl-HN-C(O)-C₁-C₆-alkyl or (C₁-C₆-alkyl)₂N-C(O)-C₁-C₆-alkyl.

R₁ and R₂ can, as alkyl, be linear or branched and preferably contain from 1 to 4 C atoms. Examples are methyl, ethyl, n- and i-propyl, n-, i- and t-butyl, pentyl and hexyl.

R₁ and R₂ can, as haloalkyl, be linear or branched and preferably contain from 1 to 4, particularly preferably 1 or 2, C atoms. Examples are fluoromethyl, difluoromethyl, trifluoromethyl, chloromethyl, dichloromethyl, trichloromethyl, 2-chloroethyl and 2,2,2-trifluoroethyl.

R₁ and R₂ can, as alkoxy, be linear or branched and preferably contain from 1 to 4 C atoms. Examples are methoxy, ethoxy, n- and i-propyloxy, n-, i- and t-butyloxy, pentyloxy and hexyloxy.

R₁ and R₂ can, as alkoxyalkyl, be linear or branched. The alkoxy group preferably contains from 1 to 4, and particularly 1 or 2, C atoms and the alkyl group preferably contains from 1 to 4C atoms. Examples are methoxymethyl, 1-methoxyeth-2-yl, 1-methoxyprop-3-yl, 1-methoxybut-4-yl, methoxypentyl, methoxyhexyl, ethoxymethyl, 1-ethoxyeth-2-yl, 1-ethoxyprop-3-yl, 1-ethoxybut-4-yl, ethoxypentyl, ethoxyhexyl, propyloxymethyl, butyloxymethyl, 1-propyloxyeth-2-yl and 1-butyloxyeth-2-yl.

R₁ and R₂ can, as C₁-C₆-alkoxy-C₁-C₆-alkyloxy, be linear or branched. The alkoxy group preferably contains from 1 to 4, and particularly 1 or 2, C atoms and the alkyloxy group preferably contains from 1 to 4 C atoms. Examples are methoxymethyloxy, 1-methoxyeth-2-yloxy, 1-methoxyprop-3-yloxy, 1-methoxybut-4-yloxy, methoxypentyloxy, methoxyhexyloxy, ethoxymethyloxy, 1-ethoxyeth-2-yloxy, 1-ethoxyprop-3-yloxy, 1-ethoxybut-4-yloxy, ethoxypentyloxy, ethoxyhexyloxy, propyloxymethyloxy, butyloxymethyloxy, 1-propyloxyeth-2-yloxy and 1-butyloxyeth-2-yloxy.

In a preferred embodiment, R₁ is methoxy- or ethoxy-C₁-C₄-alkyloxy, and R₂ is preferably methoxy or ethoxy. Very particular preference is given to compounds of formula I in which R₁ is 1-methoxyprop-3-yloxy and R₂ is methoxy.

R₃ and R₄ can, as alkyl, be linear or branched and preferably contain from 1 to 4 C atoms. Examples are methyl, ethyl, n- and i-propyl, n-, i- and t-butyl pentyl and hexyl. In a preferred embodiment, R₃ and R₄ are in each case isopropyl in the compounds of the formula I.

R₅ can, as alkyl, be linear or branched and preferably contain from 1 to 4 C atoms. Examples of alkyl have been mentioned previously. Preference is given to methyl, ethyl, n-and i-propyl, and n-, i- and t-butyl.

R₅ can, as C₁-C₆-hydroxyalkyl, be linear or branched and preferably contain from 2 to 6 C atoms. Some examples are 2-hydroxyeth-1-yl, 2-hydroxyprop-1-yl, 3-hydroxyprop-1-yl, 2-, 3-or 4-hydroxybut-1-yl, hydroxypentyl and hydroxyhexyl.

R₅ can, as C₁-C₆-alkoxy -C₁-C₆-alkyl, be linear or branched. The alkoxy group preferably contains from 1 to 4 C atoms and the alkyl group preferably contains from 2 to 4 C atoms. Some examples are 2-methoxyeth-1-yl, 2-methoxyprop-1-yl, 3-methoxyprop-1-yl, 2-, 3- or 4-methoxybut-1-yl, 2-ethoxyeth-1-yl, 2-ethoxyprop-1-yl, 3-ethoxyprop-1-yl, and 2-, 3- or 4-ethoxybut-1-yl.

R₅ can, as C₁-C₆-alkanoyloxy-C₁-C₅-alkyl, be linear or branched. The alkanoyl group preferably contains from 1 to 4 C atoms and the alkyl group preferably contains from 2 to 4 C atoms. Some examples are formyloxymethyl, formyloxyethyl, acetyloxyethyl, propionyloxyethyl and butyroyloxyethyl.

R₅ can, as C₁-C₆-aminoalkyl, be linear or branched and preferably contain from 2 to 4 C atoms. Some examples are 2-aminoethyl, 2- or 3-aminoprop-1-yl and 2-, 3- or 4-aminobut-1-yl.

R₅ can, as C₁-C₆-alkylamino-C₁-C₆-alkyl and C₁-C₆-dialkylamino-C₁-C₆-alkyl, be linear or branched. The alkylamino group preferably contains C₁-C₄-alkyl groups and the alkyl group preferably contains from 2 to 4 C atoms. Some examples are 2-methylaminoeth-1-yl, 2-dimethylaminoeth-1-yl, 2-ethylaminoeth-1-yl, 2-diethylaminoeth-1-yl, 3-methylaminoprop-1-yl, 3-dimethylaminoprop-1-yl, 4-methylaminobut-1-yl and 4-dimethylaminobut-1-yl.

R₅ can, as C₁-C₆-alkanoylamido-C₁-C₆-alkyl, be linear or branched. The alkanoyl group preferably contains from 1 to 4 C atoms and the alkyl group preferably contains from 1 to 4 C atoms. Some examples are 2-formamidoeth-1-yl, 2-acetamidoeth-1-yl, 3-propionylamidoeth-1-yl and 4-butyroylamidoeth-1-yl.

R₅ can, as HO(O)C-C₁-C₃-alkyl, be linear or branched and the alkyl group preferably contains from 2 to 4 C atoms. Some examples are carboxymethyl, carboxyethyl, carboxypropyl and carboxybutyl.

R₅ can, as C₁-C₆-alkyl-O-(O)C-C₁-C₆-alkyl, be linear or branched, and the alkyl groups preferably contain, independently of each other, from 1 to 4 C atoms. Some examples are methoxycarbonylmethyl, 2-methoxycarbonyleth-1-yl, 3-methoxycarbonylprop-1-yl, 4-methoxycarbonylbut1yl, ethoxycarbonylmethyl, 2-ethoxycarbonyleth-1-yl, 3-ethoxy-carbonylprop-1-yl and 4-ethoxycarbonylbut-1-yl.

R₅ can, as H₂N-C(O)-C₁-C₆-alkyl, be linear or branched, and the alkyl group preferably contains from 2 to 6 C atoms. Some examples are carbamidomethyl, 2-carbamidoeth-1-yl, 2-carbamido-2,2-dimethyleth-1-yl, 2- or 3-carbamidoprop-1-yl, 2-, 3- or 4-carbamidobut-1-yl, 3-carbamido-2-methylprop-1-yl, 3-carbamido-1,2-dimethylprop-1-yl, 3-carbamido-3-methylprop-1-yl, 3-carbamido-2,2-dimethylprop-1-yl, 2-, 3-, 4- or 5-carbamidopent-1-yl, or 4-carbamido-3,3- or -2,2-dimethylbut-1-yl.

R₅ can, as C₁-C₆-alkyl-HN-C(O)-C₁-C₆-alkyl or (C₁-C₆-alkyl)₂N-C(O)-C₁-C₅-alkyl, be linear or branched, and the NH-alkyl group preferably contains from 1 to 4 C atoms, and the alkyl group preferably contains from 2 to 6 C atoms. Examples are the previously mentioned carbamidoalkyl groups whose N atom is substituted by one or two methyl, ethyl, propyl or butyl.

The composition according to the invention comprises a subgroup of compounds of formula I formed by those in which R₁ is C₁-C₄₋alkoxy or C₁-C₄-alkoxy-C₁-C₄-alkyloxy, R₂ is C₁-C₄-alkoxy, R₃ is C₁-C₄-alkyl, R₄ is C₁-C₄-alkyl and R₅ is optionally N-mono- or N-di-C₁-C₄-alkyl-substituted H₂NC(O)-C₁-C₆-alkyl

A more preferred subgroup of compounds of the formula I is formed by those in which R₁ is methoxy-C₂-C₄-alkyloxy, R₂ is methoxy or ethoxy, R₃ is C₂-C₄-alkyl, R₄ is C₂-C₄-alkyl and R₅ is H₂NC(O)-C₁-C₆-alkyl.

The ω-phenyloctanecarboxamide derivative which is very particularly preferred is the compound of the formula la,

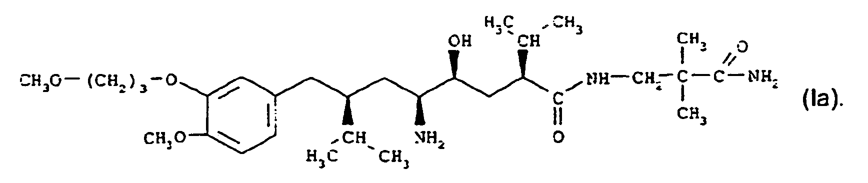

The hemifumarate of the formula Ia is termed SPP100B below.

The renin inhibitors are relatively large molecules. ω-phenyloctanecarboxamide derivatives, particularly of the formula la, are very readily soluble in water. All these properties suggest low oral bioavailability.

Physiologically tolerated anionic and neutral surfactants are known as auxiliary substances in oral formulations of pharmaceutical active compounds and are listed, for example, in the American Code of Federal Regulations Title 21 (Food and Drugs), revised 1 April 2001.

Anionic surfactants are widely known. They are mainly organic acids and their physiologically tolerated salts, such as alkali metal salts (Na or K) or alkaline earth metal salts (Mg or Ca) which contain a hydrophobic substituent. Examples of suitable acids are carboxylic acids, sulfonic acids, sulfinic acids, phosphonic acids, phosphonous acids, sulfuric acid monoesters, monoesters of sulfurous acid, phosphoric acid monoesters or diesters and monoesters or diesters of phosphorous acid, and also sulfated unsaturated carboxylic acid esters. Preferred acids are sulfated unsaturated carboxylic acid esters, sulfonic acids, phosphonic acids, sulfuric acid monoesters and phosphoric acid monoesters or diesters. Particular preference is given to sulfuric acid monoesters, dialkyl sulfosuccinates and phosphoric acid monoesters or diesters.

The acids preferably contain saturated or unsaturated hydrocarbon radicals having at least 6, preferably at least 8, C atoms and up to 30, preferably up to 20, C atoms. The hydrocarbon radicals can be interrupted by O, S, CO, -C(O)-O- and/or -C(O)-NH-, and/or be unsubstituted or substituted by -OH, -O-C₁-C₂₀-alkyl, -NH-C(O)-C₁-C₂₀-alkyl and/or -O-C(O)-C₁-C₂₀-alkyl. The hydrocarbon radicals can be selected from the group linear and branched alkyl, C₁-C₂₀-alkyl-substituted C₅-C₁₂-cycloalkyl and preferably C₅-C₈-cycloalkyl, C₁-C₂₀-alkylsubstituted C₆-C₁₀-aryl and C₅-C₁₂-cycloalkyl-substituted or C₈-C₃₀-polycycloalkyl-substituted C₁-C₂₀-alkyl. Polycycloalkyl preferably means condensed ring systems as can be found in the naturally occurring steroid and bile acids.

The anionic surfactant can correspond to the formulae II and IIa,

R-X (II),

R-C(O)-NH-R₆-SO₃H (IIa),

in which R is a saturated or unsaturated hydrocarbon radical having from 6 to 30 C atoms which is optionally interrupted by -O-, -S-, -CO-, -C(O)-O- and/or -C(O)-NH-, and/or is unsubstituted or substituted by -OH, -O-C₁-C₂₀-alkyl, -NH-C(O)-C₁-C₂₀-alkyl and/or -O-C(O)-C₁-C₂₀-alkyl, R₆ is C₂-C₄-alkylene and X is -SO₃H, -COOH or -OSO₃H, and also their sodium, potassium, magnesium and calcium salts. Examples of surfactants of the formula II are C₆-C₂₀-monoalkyl sulfates such as octyl sulfate, decyl sulfate, dodecyl sulfate, tetradecyl sulfate, hexadecyl sulfate and octadecyl sulfate, and also salts of fatty acids (Na oleate or Na caprate). Examples of surfactants of the formula IIa are 1-acylaminoethane-2-sulfonic acids, such as 1-octanoylaminoethane-2-sulfonic acid, 1-decanoylaminoethane-2-'sulfonic acid, 1-dodecanoylaminoethane-2-sulfonic acid, 1-tetradecanoylaminoethane-2-sulfonic acid, 1-hexadecanoylaminoethane-2-sulfonic acid, and 1-octadecanoylaminoethane-2-sulfonic acid, and taurocholic acid and taurodeoxycholic acid. Bile acids and their salts, such as cholic acid, deoxycholic acid and sodium glycocholates, are also suitable.

Other suitable anionic surfactants are semiesters composed of polycarboxylic acids, such as malonic acid, succinic acid, glutaric acid, adipic acid, maleic acid and fumaric acid, and C₆-C₂₀-alkanols or C₆-C₂₀-alkenols, and their salts, for example sodium stearylsuccinate.

Particularly preferred anionic surfactants are alkali (alkaline earth) metal salts of saturated fatty acids such as sodium caprate or sodium laurate and unsaturated fatty acids, such as sodium oleate as well as alkyl sulfates, such as sodium lauryl sulfate and sodium cetyl sulfate. Other examples of particularly preferred compounds are sulfated castor oil and sodium dioctylsulfosuccinate.

Amphoteric surfactants are also suitable; among these, preference is given to natural or modified lecithins and phospholipids. The lecithins can be natural, partially hydrogenated or hydrogenated lecithins or sphingolipids. Natural lecithins are mixtures of different phospholipids. Examples of phospholipids are phosphatidylcholine, phosphatidyl ethanolamine, lysophosphatidyl choline, phosphatidyl glycerol, phosphatidic acid and phosphatidyl serine and their partially hydrogenated or completely hydrogenated derivatives. Examples of phospholipids containing defined fatty acids are 1,2-dimyristoyl-sn-glycero-3-phosphocholine, 1,2-dipalmitoyl-sn-glycero-3-phosphocholine, 1,2-distearoyt-sn-glycero-3-phosphocholine, 1,2-dioleoyl-sn-glycero-3-phosphocholine, 1-palmitoyl-2-oleoyl-sn-glycero-3-phosphocholine, 1,2-dimyristoyl-sn-glycero-3-phospho-rac-glycerol, 1,2-dipalmitoyl-sn-glycero-3-phospho-rac-glycerol and 1,2-distearoyl-sn-glycero-3-phospho-rac-glycerol. Preference is given to using lecithin and phosphatidyl choline

Examples of other known amphoteric surfactants are N-mono- or -dialkylated aminocarboxylic acids (betaines), with it being possible for the alkyl group to contain from 6 to 30, preferably from 8 to 20, C atoms. Examples are cocamidopropylbetaine and laurylbetaine (Amphoteen^{®} 24). Other amphoteric surfactants are known from the class of the aminocarboxylic acids and their salts, and also as derivatives of imidazolines.

Natural lecithin is a preferred amphoteric surfactant.

Neutral surfactants are also known. These can, for example, be fatty alcohols and cholesterols, which are frequently used in combination with alkyl sulfates or polyethylene glycol monoalkyl esters.

Other known surfactants are monoesters or diesters composed of glycerol and C₈-C₃₀₋carboxylic acids, in particular fatty acids, for example glycerol mono- or -distearate, glycerol mono- or -dioleate and glycerol mono- or -dipalmitate. Another group are ethoxylated partial fatty acid esters composed of polyols, such as ethylene glycol, propylene glycol, glycerol or pentaerythritol, and optionally hydrogenated polyoxy castor oils which can be obtained commercially as Chremophors®. Chremophor® EL and Chremophor® RH40 are preferred surfactant types.

Suitable neutral surfactants are also partial fatty acid esters of sorbitan which can be obtained commercially as SPAN® or ARLACEL®, and also partial fatty acid esters of sucrose.

Other suitable surfactants are fatty acid esters of polyols, such as ethylene glycol or pentaerythrol or polyethylene glycols, such as polyoxyethylene stearate, which esters can be obtained commercially in various types, for example as Myrj®.

Known neutral surfactants are also fatty alcohol ethers of polyoxyethylene, for example lauryl-, myristyl-, cetyl- and oleylpolyoxyethylene ethers. These can be obtained commercially in various types, for example as Brij®.

Sorbitan-based ethoxylated partial fatty acid esters are also known to be surfactants, with these surfactants being termed polysorbates and being offered for sale commercially in various types, for example as TWEEN®

Finally, polyethylene polypropylene glycols should also be mentioned. These surfactants are block copolymers containing blocks composed of polyoxyethylene and polyoxypropylene, with these block copolymers being termed poloxamers and being available commercially as Pluronics®. The blocks can be of varying chain length and the substances can be liquid to solid. Polyoxyethylene blocks can, for example, contain from 5 to 120, preferably from 10 to 100, oxyethylene units and polyoxypropylene blocks can contain from 10 to 80, and preferably from 10 to 50, oxypropylene units. The chain lengths of the blocks and the molecular weight of the substance can be used to achieve desired properties in a selective manner. Poloxamers 124, 188 and 407 are preferred examples.

Preferred neutral surfactants are block copolymers composed of polyoxyethylene and polyoxypropylene, partial fatty acid esters of sorbitan, ethoxylated partial fatty acid esters of sorbitan, fatty alcohol ethers and fatty acid esters of polyoxyethylenes and hydrogenated, polyethoxylated castor oils.

Suitable surfactants are described, for example, in pharmacopoeias such as USP25/NF20 or can be identified from the literature, in this present case for example, from Martindale, thirty-second edition 1999, pages 1324-1329 and 1468-1469.

The composition according to the invention can be produced in a simple manner by mixing the components. The composition can be liquid to oily, semisolid or solid. The consistency of the composition depends essentially on the choice of surfactant or combination of surfactants and the quantitative composition. Known methods for mixing the components are dry mixing of pulverulent components, melting methods, and solution methods, involving dissolving the components and subsequently removing the solvents.

Solvents are expediently selected such that they can be removed virtually completely. Suitable solvents are water and organic solvents, particularly polar organic solvents, which can also be used as mixtures of at least two solvents. Examples of pharmaceutically customary solvents are halohydrocarbon (methylene chloride); ketones (acetone); alcohols (methanol. ethanol, n- or i-propanol, or n- or i-propanediol); nitriles (acetonitrile); and tertiary amines (N-methylpyrrolidine).

Because of its increased bioavailability, the composition according to the invention is outstandingly suitable for producing forms for oral administration. Because of the increased bioavailability, it is possible to provide doses which are physiologically harmless as far as the active compound and surfactant are concerned.

The invention also relates to an oral dosage form which comprises a composition according to the invention.

Examples of oral dosage forms are tablets or sugar-coated tablets, capsules composed of hard or soft gelatin or starch, and potable preparations.

Depending on the intended therapy, i.e. single or repeated consecutive and chronologically delayed administration, the oral administration form can comprise a renin inhibitor in quantities of from 10 to 600 mg, preferably of from 30 to 300 mg, and in particular of from 50 to 200 mg.

The skilled person is familiar with the production of tablets, sugar-coated tablets, capsules and potable preparations and the auxiliary substances which are required for this purpose.

Potable preparations principally comprise water. In addition, they can comprise physiologically tolerated solvents, for example alkanols such as ethanol. Customary thickeners can be used in order to stabilize suspensions.

Capsules can be filled directly with the composition according to the invention. However, the composition of the material which is used to fill the capsules can also comprise customary pharmaceutical auxiliary substances such as fillers, binders, disintegrants, lubricants and flavourings.

It is possible to use customary auxiliary substances, such as binders, fillers, lubricants and flavourings, for formulating tablets and sugar-coated tablets. These auxiliary substances are known and are therefore not described in detail.

All the solid administration forms can be provided with a costing of any given functionality. Pharmaceutically customary auxiliary substances, such as semisynthetic or fully synthetic film-forming agents, and suitable additives, such as plasticizers and dye pigments, have also to be provided for this purpose.

The invention also relates to a method for increasing the bioavailability of non-peptide renin inhibitors which is characterized in that the said renin inhibitor is mixed with at least one physiologically tolerated anionic surfactant or at least one physiologically tolerated neutral or amphoteric surfactant or with a mixture consisting of at least two of these surfactants.

The composition according to the invention makes it possible to produce oral forms for administering renin inhibitors which, because of an increased bioavailability, can be used for higher dosages and are practical for a patient.

The following examples explain the invention in more detail.

### A) Producing compositions

### Example A1: Producing a powder mixture

75 g of SPP1 00B and 75 g of sodium lauryl sulfate are weighed into a mixing box and mixed for 10 minutes in a Turbula mixer. The resulting mixture is brushed through a sieve having a mesh aperture of 0.5 mm. The sieve mixture is then once again agitated for 10 minutes in the Turbula mixer.
The desired quantities of these powders are apportioned and aliquoted, for example, into bottles. Prior to administration, the preparation is dissolved in water or another suitable physiologically well-tolerated liquid.

### Example A2: Solution method

75 g of SPP100B are kneaded with 7.5 g of sorbitan monooleate and 15 g of Polysorbat 80, dissolved in 15 ml of 99% ethanol. The resulting mass is dried at 50°C in vacuo until a constant weight is reached. Portions depending on the dose are packaged into suitable receptacles or processed to produce oral administration forms.

### B) Producing oral administration forms

### Example B1: Producing hard gelatin capsules

One capsule contains:

| | |
|---|---|
| SPP100B | 83 mg |
| Microcrystalline cellulose | 95 mg |
| Crospovidone (a polyvinylpyrrolidone) | 26 mg |
| Colloidal silicon dioxide | 2 mg |
| Sodium lauryl sulfate | 30 mg |
| Magnesium stearate | 4 mg |

The active compound, the filler, the disintegrant, the flow regulating agent and the surfactant are mixed in one operational step. The mixture is sieved and mixed once again in the dry. Finally, magnesium stearate is added as lubricant and admixed for 3 minutes. In conclusion, the mass, corresponding to 240 mg, is aliquoted into size 0 capsules.

### Example B2: Producing soft gelatin capsules

One capsule contains:

| | |
|---|---|
| SPP100B | 75 mg |
| Hydrogenated vegetable oil | 50 mg |
| Medium-chain triglycerides (MCT) | 250 mg |
| Lecithin | 150 mg |
| Glycerol stearate | 50 mg |
| Yellow wax | 30 mg |
| Oleic acid | 10 mg |
| Ascorbyl palmitate | 5 mg |

All the auxiliary substances are weighed into a glass vessel. The mass is heated and stirred until a clear solution is obtained. The melt is then homogenized for 10 minutes. SPP100B is added and the mass is brought to a suitable temperature for aliquoting, while being subjected to further stirring and homogenization, and encapsulated in soft gelatin.

### C) Application examples:

### Example C1: Determining the bioavailability

The bioavailability of a powder mixture composed of surfactant and active compound is compared with that of SPP100B on its own in an absorption study carried out in rats. The rat model is chosen since, in this model, the absorption of the active compound is low and small quantities of active compound can be investigated.

The active compound, or a mixture of 2 parts of SPP100B and 1 part of sodium lauryl sulfate, is in each case administered to 10 rats. The plasma levels are measured over a period of 24 hours after administering the dose. In this model, it is found that adding this surfactant to the renin inhibitor significantly increases oral bioavailability.

## Claims

1. Composition comprising (1) a non-peptide renin inhibitor of formula I 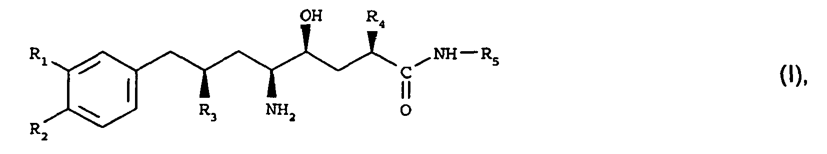 in which
R₁ is C₁-C₄-alkoxy or C₁-C₄-alkoxy-C₁-C₄-alkyloxy,
R₂ is C₁-C₄-alkoxy,
R₃ is C₁-C₄-alkyl,
R₄ is C₁-C₄-alkyl and
R₅ is optionally N-mono- or N-di-C₁-C₄-alkyl-substituted H₂NC(O)-C₁-C₆-alkyl and (2) at least one physiologically tolerated anionic surfactant, with the quantity of the renin inhibitor being at least 10% by weight, based on the composition.

2. Composition according to claim 1, **characterized in that** the renin inhibitor is a compound of the formula Ia or its physiologically tolerated salts

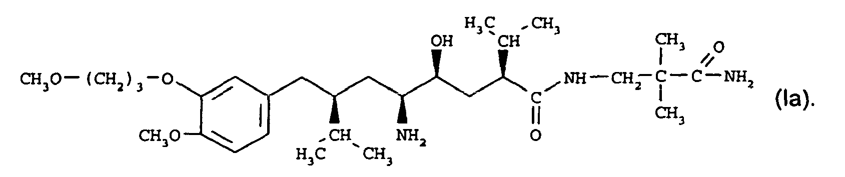

3. Composition according to claim 1 **characterized in that** the quantity of the renin inhibitor is from 10 to 90% by weight, based on the composition.

4. Composition according to claim 3, **characterized in that** the quantity of the renin inhibitor is from 50 to 90% by weight, based on the composition.

5. Composition according to claim 1, **characterized in that** the anionic surfactants are organic acids, and their physiologically tolerated salts of alkali metals or alkaline earth metals, which contain a hydrophobic substituent.

6. Composition according to claim 5, **characterized in that** it is sodium lauryl sulfate, sodium cetyl sulfate, sulfated castor oil or sodium dioctyl sulfosuccinate.

7. Oral administration form comprising a composition according to claim 1.

8. Oral administration form according to claim 7, **characterized in that** it comprises tablets, sugar-coated tablets, capsules or a potable preparation.

9. Oral administration form according to claim 7, **characterized in that** the renin inhibitor is present in a quantity of from 10 to 600 mg, based on the administration form.

10. Method for increasing the bioavailability of a non-peptide renin inhibitor of formula I 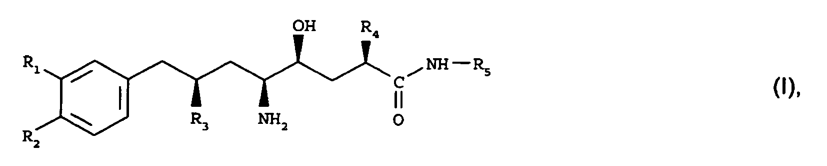 in which
R₁ is C₁-C₄-alkoxy or C₁-C₄-alkoxy-C₁-C₄-alkyloxy,
R₂ is C₁-C₄-alkoxy,
R₃ is C₁-C₄-alkyl,
R₄ is C₁-C₄-alkyl and
R₅ is optionally N-mono- or N-di-C₁-C₄-alkyl-substituted H₂NC(O)-C₁-C₆-alkyl **characterized in that** the said renin inhibitor is mixed with at least one physiologically tolerated anionic surfactant.

## Patentansprüche

1. Zusammensetzung, enthaltend (1) einen nicht-peptidischen Renin-Inhibitor der Formel 1 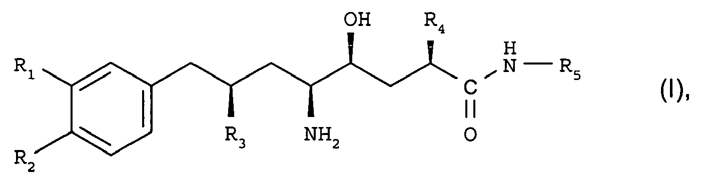 worin
R₁ C₁-C₄-Alkoxy oder C₁-C₄-Alkoxy-C₁-C₄-Alkyloxy,
R₂ C₁-C₄-Alkoxy,
R₃ C₁-C₄-Alkyl,
R₄ C₁-C₄-Alkyl und
R₅ optional N-mono- oder N-di-C₁-C₄-Alkyl substituiertes H₂N-C(O)-C₁-C₆-Alkyl bedeutet, und (2) wenigstens ein physiologisch verträgliches anionisches Tensid,
wobei die Menge des Renin-Inhibitors wenigstens 10 Gew.-% beträgt, bezogen auf die Zusammensetzung.

2. Zusammensetzung gemäss Anspruch 1, **dadurch gekennzeichnet, dass** es sich bei dem Renin-Inhibitor um eine Verbindung der Formel Ia oder deren physiologisch verträgliche Salze handelt:

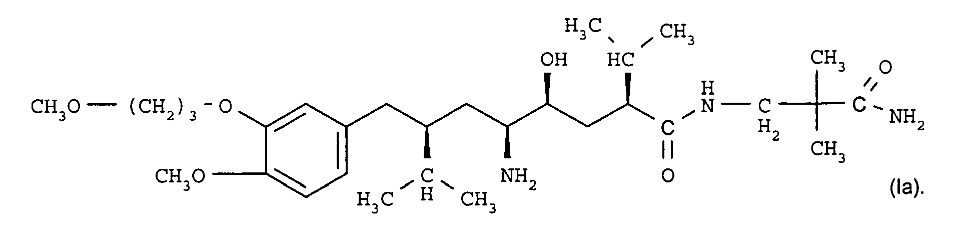

3. Zusammensetzung gemäss Anspruch 1, **dadurch gekennzeichnet, dass** die Menge an Renin-Inhibitor 10 bis 90 Gew.-% beträgt, bezogen auf die Zusammensetzung.

4. Zusammensetzung gemäss Anspruch 3, **dadurch gekennzeichnet, dass** die Menge an Renin-Inhibitor 50 bis 90 Gew.-% beträgt, bezogen auf die Zusammensetzung.

5. Zusammensetzung gemäss Anspruch 1, **dadurch gekennzeichnet, dass** es sich bei den anionischen Tensiden um organische Säuren und deren physiologisch verträglichen Salze von Alkalimetallen oder Erdalkalimetall handelt, die einen hydrophoben Substituenten enthalten.

6. Zusammensetzung gemäss Anspruch 5, **dadurch gekennzeichnet, dass** es sich um Natriumlaurylsulfat, Natriumcetylsulfat, sulphatiertes Rizinusöl, Natriumdioctylsulfosuccinat handelt.

7. Orale Darreichungsform, enthaltend eine Zusammensetzung gemäss Anspruch 1.

8. Orale Darreichungsform gemäss Anspruch 7, **dadurch gekennzeichnet, dass** es sich um Tabletten, Dragées, Kapseln oder eine trinkbare Zubereitung handelt.

9. Orale Darreichungsform gemäss Anspruch 7, **dadurch gekennzeichnet, dass** der Renin-Inhibitor in einer Menge von 10 bis 600 mg enthalten ist, bezogen auf die Darreichungsform.

10. Verfahren zur Erhöhung der Bioverfügbarkeit eines nicht-peptidischen Renin-Inhibitors der Formel I 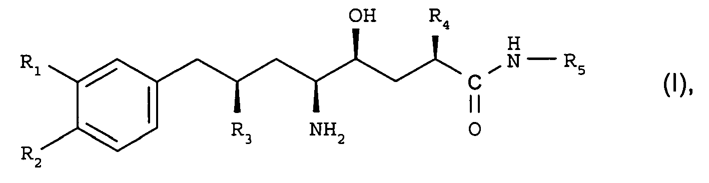 worin
R₁ C₁-C₄-Alkoxy oder C₁-C₄-Alkoxy-C₁-C₄-Alkyloxy,
R₂ C₁-C₄-Alkoxy,
R₃ C₁-C₄-Alkyl,
R₄ C₁-C₄-Alkyl und
R₅ optional N-mono- oder N-di-C₁-C₄-Alkyl substituiertes H₂N-C(O)-C₁-C₆-Alkyl bedeutet, **dadurch gekennzeichnet, dass** man den besagten Renin-Inhibitor mit wenigstens einem physiologisch verträglichen anionischen Tensid vermischt.

## Revendications

1. Composition comprenant (1) un inhibiteur de rénine non peptidique de formule I 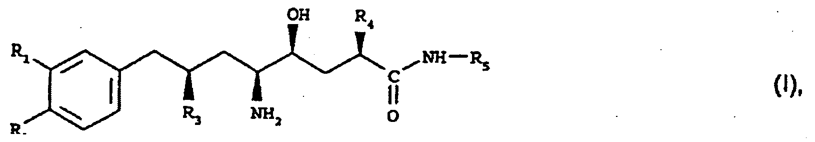 dans laquelle
R₁ est un radical alcoxy en C₁-C₄ ou (alcoxy en C₁₋C₄) (alkyloxy en C₁-C₄) ,
R₂ est un radical alcoxy en C₁-C₄,
R₃ est un radical alkyle en C₁-C₄,
R₄ est un radical alkyle en C₁-C₄, et
R₅ est un radical H₂NC(O)-(alkyle en C₁-C₆), éventuellement N-mono- ou N-di-(alkyle en C₁-C₄)-substitué,
et (2) au moins un tensioactif anionique toléré d'un point de vue physiologique, la quantité de l'inhibiteur de rénine étant d'au moins 10 % en poids par rapport à la composition.

2. Composition selon la revendication 1, **caractérisée en ce que** l'inhibiteur de rénine est un composé de formule Ia, ou ses sels tolérés d'un point de vue physiologique

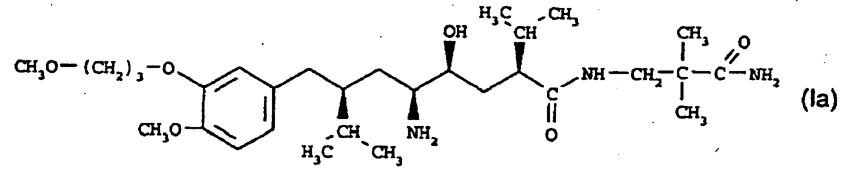

3. Composition selon la revendication 1, **caractérisée en ce que** la quantité de l'inhibiteur de rénine est de 10 à 90 % en poids par rapport à la composition.

4. Composition selon la revendication 3, **caractérisée en ce que** la quantité de l'inhibiteur de rénine est de 50 à 90 % en poids par rapport à la composition.

5. Composition selon la revendication 1, **caractérisée en ce que** les tensioactifs anioniques sont des acides organiques, et leurs sels tolérés d'un point de vue physiologique de métaux alcalins ou de métaux alcalino-terreux, qui contiennent un substituant hydrophobe.

6. Composition selon la revendication 5, **caractérisée en ce qu'**il s'agit du laurylsulfate de sodium, du cétylsulfate de sodium, de l'huile de ricin sulfatée ou du dioctylsulfosuccinate de sodium.

7. Forme pour administration orale comprenant une composition selon la revendication 1.

8. Forme pour administration orale selon la revendication 7, **caractérisée en ce qu'**elle comprend des comprimés, des comprimés dragéifiés, des gélules ou une préparation buvable.

9. Forme pour administration orale selon la revendication 7, **caractérisée en ce que** l'inhibiteur de rénine est présent en une quantité de 10 à 600 mg par rapport à la forme d'administration.

10. Procédé pour augmenter la biodisponibilité d'un inhibiteur de rénine non peptidique de formule I 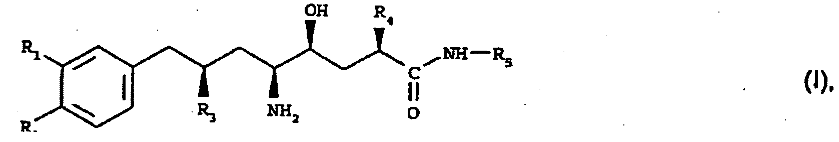 dans laquelle
R₁ est un radical alcoxy en C₁-C₄ ou (alcoxy en C₁₋C₄) (alkyloxy en C₁-C₄),
R₂ est un radical alcoxy en C₁-C₄,
R₃ est un radical alkyle en C₁-C₄,
R₄ est un radical alkyle en C₁-C₄, et
R₅ est un radical H₂NC(O)-(alkyle en C₁-C₆), éventuellement N-mono- ou N-di-(alkyle en C₁-C₄)-substitué,
**caractérisé en ce que** ledit inhibiteur de rénine est mélangé à au moins un tensioactif anionique toléré d'un point de vue physiologique. .
